# DEMANDE DE BREVET EUROPEEN

(11) **EP 0 774 253 A1**
(43) Date de publication de la demande: **21.05.1997**
(21) Numéro de dépôt: 96402580.3
(22) Date de dépôt: 28.11.1996
(51) Int. Cl.: A61K 31/165, A61K 9/20

(54) **Nouvelles compositions pharmaceutiques à action anti-migraineuse contenant du paracetamol et du metoclopramide**

(30) Priorité: 28.11.1995 FR 9514043
(71) Demandeur: BOUCHARA S.A., F-92300 Levallois-Perret (FR)
(72) Inventeur: Santini, Claude, 92190 Meudon (FR)
(74) Mandataire: Burtin, Jean-François

(57) **Abrégé**

L' invention se rapporte au domaine de la chimie thérapeutique et plus particulièrement à celui de la pharmacie galénique.

Elle a spécifiquement pour objet de nouvelles compositions pharmaceutiques caractérisées en ce qu'elles renferment comme principes actifs du paracetamol et du metoclopramide ou un de ses sels en association avec un ou plusieurs excipients ou véhicules inertes non toxiques, pharmaceutiquement acceptables.

Les compositions pharmaceutiques selon l'invention conviennent comme médicament pour le soulagement de la migraine.

## Description

La présente invention se rapporte au domaine de la chimie thérapeutique et plus particulièrement au domaine de la pharmacie galénique.
La présente invention se rapporte plus particulièrement à de nouvelles compositions pharmaceutiques destinées au traitement de la migraine formées d'un agent antalgique et d'un agent antidopaminergique.

Elle a plus spécifiquement pour objet de nouvelles compositions pharmaceutiques destinées au soulagement de la migraine caractérisées en ce qu'elles contiennent comme principes actifs du paracétamol et du métoclopramide ou un de ses sels.
On sait combien le traitement de la migraine est difficile étant donné la multiplicité des formes et des causes déclenchant la migraine. Il existe des causes vasculaires, il existe des causes alimentaires, il existe des causes tensionnelles entre autres pour déclencher la migraine. De ce fait les médicaments qui empêchent la migraine ou qui soulagent les patients atteints de migraine sont nombreux mais peu sont suffisamment efficaces. Les alcaloïdes de l'ergot de seigle et les préparations qui en contiennent sont efficaces sur certaines formes de migraine, les anti-histaminiques donnent des résultats sur d'autre formes de migraine, certaines substances antisérotoninergiques donnent aussi des résultats sur d'autres formes de migraine. Mais peu de ces médicaments sont déjà actifs pour atténuer ou supprimer la sensation douloureuse, en particulier la douleur intense et continue au niveau des tempes ou de la partie frontale.

C'est pourquoi il était souhaitable de rechercher un médicament qui soit actif sur l'élément douleur sans provoquer les effets dépresseurs ou hypnotiques liés aux antalgiques centraux ou aux anti-histaminiques.

Cet objectif a pu être atteint grâce aux compositions selon l'invention qui manifestent une action antalgique nette chez les sujets migraineux sans toutefois déclencher des effets de somnolence de vertige ou de dépression liés à l'utilisation d'antalgiques centraux.

L'association de paracétamol et d'une substance antidopaminergique a permis de réaliser cet objectif thérapeutique. La substance antidopaminergique la plus appropriée est le métoclopramide ou un de ses sels (mono ou di-sel) dont l'effet sur le système nerveux central est limité et qui manifeste en outre un effet antinauséeux utile pour le soulagement des états migraineux.

Les compositions pharmaceutiques selon l'invention se caractérisent donc par le fait qu'elles contiennent une dose unitaire de paracétamol allant de 0,500g à 1,000g et une dose de métoclopramide, ou d'un de ses sels, allant de 0,0075g à 0,015g en association ou en mélange avec un ou plusieurs excipients - ou véhicules inertes - non-toxiques pharmaceutiquement acceptable.

De préférence les compositions pharmaceutiques selon l'invention contiennent 1g de paracétamol et 0,010g de métoclopramide. Les compositions sont destinées à être administrées par voie digestive, sous-cutanée, rectale, perlinguale, transcutanée ou pernasale.

Ainsi il sera possible de les présenter sous forme :
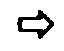 de préparations sèches - comme des comprimés nus ou enrobés, de dragées, de pilules, de sachets de poudre, de gélules ou de capsules pour l'administration orale,
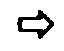 de solutions ou de suspensions ou d'émulsions aqueuses pour la voie sous-cutanée,
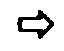 de suppositoires ou de capsules pour la voie rectale,
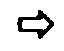 de comprimés ou de tablettes sublinguales, de solutions ou de suspensions pour l'utilisation par voie transcutanée,
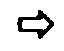 de solutions ou de suspensions pour l'utilisation par voie pernasale, additionnées ou non de composés facilitant le passage à travers les muqueuses comme l'éthylène glycol, le propylène glycol ou le diméthyl isosorbide.

Les formes destinées à la voie orale comme les dragées, les comprimés nus et les comprimés enrobés sont préparées selon un procédé qui consiste à incorporer les principes actifs dans un ou plusieurs excipients comme des diluants, agents liants, des agents de compression ou des agents d'éclatement. Le mannitol, le lactose, les amidons, les celluloses, les alcoylcelluloses. les sels minéraux insolubles comme les carbonates de métal alcalino-terreux, les phosphates de métal alcalino-terreux, le dioxyde de titane, les stéarates de métal alcalino-terreux, les silicates naturels ou artificiels. les lécithines ou l'urée en sont des exemples.

Les compositions selon l'invention peuvent également se présenter sous forme lyophilisée constituée par : une quantité déterminée de chacun des constituant actifs comprise entre 30 et 80% de la masse sèche du lyophilisat, un diluant capable d'améliorer les propriétés physiques de la dite forme pharmaceutique et pouvant représenter jusqu'à 70% de la masse sèche lyophilisée, un ou plusieurs agents liants constituant au total de 0,1 à 10% de la masse sèche lyophilisée et un ou plusieurs additifs destinés à améliorer le goût et/ou à modifier la couleur et/ou à améliorer la conservation.

On entend par agent liant toute matière soluble ou dispersible dans l'eau acceptable du point de vue pharmaceutique et inerte vis à vis des deux principes actifs. Ces matières sont notamment choisies de manière à assurer au produit final lyophilisé une dureté suffisante pour permettre l'extrusion à travers un blister, et un bon délitement. Généralement les agents liants sont choisis parmi les dextranes, les dérivés hydrodispersibles de l'amidon, les silices colloïdales, les celluloses, les gommes naturelles ou synthétiques, la polyvinyl-pyrrolidone, l'alcool polyvinylique, les polyéthylèneglycols (PEG 20 000, PEG 6 000), ou encore des mélanges de matières citées ci-dessus.

En outre, la composition pharmaceutique peut contenir d'autres additifs comme des colorants, des substances modifiant le goût, des agents conservateurs, ou toute autre substance compatible avec le reste du mélange.

Les substances modifiant le goût peuvent être tout produit habituellement utilisé dans l'industrie alimentaire ou pharmaceutique. Par exemple les arômes (arôme orange, arôme citron...), le saccharose, le glucose, le xylose, le sorbitol, le mannitol, le xylitol, la saccharine, les saccharinates, les cyclamates, l'aspartame, le glycyrrhizinate d'ammonium, ou les acides citrique, ascorbique ou tartrique ou encore des mélanges de ces substances.

Les agents colorants et les agents conservateurs sont ceux qui sont habituellement utilisés dans l'industrie pharmaceutique et alimentaire.

Les agents de surface ne sont pas toujours indispensables néanmoins en fonction des autres matières constituant la composition il peut parfois être utile de les ajouter.

On choisira avantageusement des agents non-ioniques [polysorbates polyoxy-éthylèniques (Tween), monopalmitate de saccharose, esters de sorbitane (Span), copolymères d'oxyde de propylène et d'éthylène, éthers de polyoxyéthylèneglycols et d'alcool gras...], les agents anioniques (esters de l'acide sulfosuccinique : sulfosuccinates d'alcoyle par exemple le dioctylsulfosuccinate de sodium), les agents cationiques (sels d'ammonium quaternaires).

La composition pharmaceutiques unitaire lyophilisée selon l'invention est tout à fait indiquée pour les traitements ambulatoires et plus spécialement encore dans le cas de sujet ayant des difficultés à déglutir ou à avaler des comprimés de dimensions importantes.

D'une manière générale les compositions préférées sont constituées par :
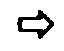 une quantité des deux principes actifs comprise entre 40 et 80% de la masse sèche du lyophilisat,
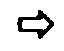 un diluant pouvant constituer jusqu'à 60% de la masse sèche lyophilisée,
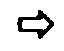 les autres additifs cités précédemment.

La présente invention concerne également la préparation de la nouvelle forme pharmaceutique selon l'invention selon les opérations suivantes:
1) préparation d'une pâte contenant les différents constituants énumérés ci-dessus, ainsi qu'une quantité d'eau convenable de manière à ajuster la viscosité de la suspension obtenue,
2) division de la pâte en quantités unitaires de forme et de volume prédéterminés,
3) lyophilisation.

Il est entendu que les différents constituants peuvent être mélangés dans n'importe quel ordre, le mélange sec des constituants pouvant être préparé préalablement à l'addition de l'eau .

Par ailleurs, il est aussi entendu que la division du produit peut également être effectuée après la lyophilisation, mais il est préférable de répartir la pâte dans des alvéoles de forme et de dimensions prédéterminées, préalablement à l'opération de lyophilisation. La forme et les dimensions des alvéoles ainsi que les quantités des constituants de la pâte sont calculées de manière à obtenir une quantité précise de principes actifs dans chaque dose unitaire.

La quantité d'eau introduite pour constituer la pâte à lyophiliser est déterminée de telle manière que la suspension obtenue possède des caractéristiques rhéologiques permettant une bonne division du produit (écoulement, homogénéité du mélange, régularité du volume divisé, stabilité de la suspension pendant la division).
La quantité d'eau pourra le plus souvent varier dans des proportions allant de 20% à 60% de la préparation à lyophiliser.

Les exemples suivants illustrent la présente invention.

### EXEMPLE 1

### Préparation d'une forme lyophilisée de paracétamol et de métoclopramide.

On dissout sous agitation 0,750 kg de dextrane 70 et 0,070 kg de gomme xanthane dans 50 kg d'eau purifiée, puis on ajoute successivement, sous agitation, 0,630 kg de saccharine sodique puis 0,720 kg d'acide citrique monohydraté.
Dans un mélangeur planétaire on introduit 40,958 kg de mannitol puis 1000 kg de paracétamol et 10 kg de métoclopramide 3,500 kg d'arôme orange et 2,500 kg d'arôme citron. L'ensemble des poudres est mis sous agitation pendant 15 minutes, sous pression réduite (80 kPA).

La solution préparée précédemment est incorporée dans le mélange des poudres, puis le mélange est additionné de 0,520 kg de colorant dilué avec 1,000 kg d'eau purifiée. La suspension concentrée ainsi obtenue est divisée par une diviseuse CITUS, dans des alvéoles de chlorure de polyvinyle de 1,6 cm³.

La feuille alvéolée de chlorure de polyvinyle, contenant la suspension, est introduite dans un lyophilisateur immédiatement après division, pour être congelée une température de -30°C sous pression atmosphérique, pendant environ 2 heures. Après dessication pendant environ 12 heures sous pression réduite, la température est remontée progressivement à 30°C par paliers de 5°C.

Les lyophilisats obtenus sont directement conditionnés par thermo-scellage d'un film d'aluminium suffisamment fin pour permettre l'extrusion hors des alvéoles sans effritement.

Chaque lyophilisat unitaire contient 1000 mg de paracétamol et 10 mg de métoclopramide et donne un temps de délitement dans l'eau inférieur à 5 minutes.

### EXEMPLE 2

En opérant comme décrit à l'exemple 1, mais en utilisant 68 kg d'eau purifiée pour la constitution de la pâte, on prépare des lyophilisats ayant la composition unitaire suivante :
◇ Paracétamol 760 mg
◇ Métoclopramide dichlorhydrate 12,5 mg
◇ Dextrane 70 10,0 mg
◇ Gomme xanthane 0,7 mg
◇ Saccharine sodique 6,3 mg
◇ Acide citrique 7,2 mg
◇ Mannitol 230,0 mg
◇ Arôme orange 35,0 mg
◇ Arôme citron 25,0 mg
◇ Colorant 5,2 mg

Le temps de délitement du lyophilisat obtenu est inférieur à 5 minutes.

### EXEMPLE 3

En opérant comme décrit précédemment à l'exemple 1, mais à partir de 1,45 kg d'Aérosil 200 à la place de la gomme xanthane, en ajoutant 0,36 kg de monopalmitate de saccharose dans la solution de départ et en utilisant 38,4 kg d'eau purifiée pour la constitution de la pâte, on prépare des lyophilisats ayant la composition unitaire suivante:
◇ Paracétamol 750 mg
◇ Métoclopramide dichlorhydrate 12,5 mg
◇ Dextrane 70 21,0 mg
◇ Aérosol 200 1,45 mg
◇ Saccharine sodique 3,0 mg
◇ Acide citrique 5,0 mg
◇ Mannitol 338,0 mg
◇ Arôme orange 20,0 mg
◇ Monopalmitate de saccharose 36,0 mg
◇ Colorant 3,6 mg

Le temps de délitement du lyophilisat obtenu est inférieur à 5 minutes.

## Revendications

1. Compositions pharmaceutiques anti-migraineuses caractérisées en ce qu'elles renferment à titre de principes actifs du paracétamol et du métoclopramide ou un de ses sels, en association ou en mélange avec un ou plusieurs excipients ou véhicules inertes, non-toxiques pharmaceutiquement acceptables.

2. Compositions pharmaceutiques anti-migraineuses selon la revendication 1 caractérisées en ce qu'elles contiennent par dose unitaire de 0,500 à 1,00 g de paracétamol et de 0,0075 à 0,015g de métoclopramide ou d'un de ses sels.

3. Compositions pharmaceutiques anti-migraineuses selon la revendication 1 ou la revendication 2 caractérisées en ce qu'elles contiennent 1 g de paracétamol et 0,010 g de métoclopramide par prise unitaire.

4. Compositions pharmaceutiques anti-migraineuses selon les revendications 1 à 3 dans lesquelles l'excipient ou le véhicule est un de ceux qui conviennent pour l'administration par voie digestive, ou sous-cutanée, ou rectale, ou perlinguale, ou transcutanée ou pernasale.

5. Compositions pharmaceutiques selon les revendications 1 à 4 dans lesquelles l'excipent ou le véhicule est destiné à la réalisation de formes sèches pour la voie orale.

6. Compositions pharmaceutiques selon les revendications 1 à 5 caractérisées en ce qu'elles se présentent sous forme lyophilisée contenant outre les principes actifs, un diluant capable d'améliorer les propriétés physiques de la composition, un ou plusieurs agents liants et un ou plusieurs additifs destinés améliorer le goût et/ou à modifier la couleur et/ou à améliorer la conservation.

7. Procédé d'obtention de compositions pharmaceutiques anti-migraineuses selon l'une des revendications 1 à 6, qui consiste à incorporer le paracétamol et le métoclopramide ou un de ses sels dans un ou plusieurs diluants et/ou agents liants et/ou agents de compression et/ou agents d'éclatement, en vue de la réalisation d'une forme pharmaceutique sèche.
